# EUROPÄISCHE PATENTANMELDUNG

(11) **EP 1 380 838 A2**
(43) Veröffentlichungstag der Anmeldung: **14.01.2004**
(21) Anmeldenummer: 03015878.6
(22) Anmeldetag: 11.07.2003
(51) Int. Cl.: G01N 33/497

(54) **Gasanalyseverfahren zur Bestimmung der Reaktion eines biologischen Systems**

(30) Priorität: 11.07.2002 DE 10231541
(71) Anmelder: Gäbler, Ralph, 86559 Adelzhausen (DE)
(72) Erfinder: Gäbler, Ralph, 86559 Adelzhausen (DE)
(74) Vertreter: Stute, Ivo, Dipl.-Ing.

(57) **Zusammenfassung**

Als Verfahren zur Bestimmung der Reaktion mindestens eines biologischen Systems auf mindestens einen exogen Einfluss, das eine genauere Möglichkeit zur Untersuchung der Reaktion von biologischen Systemen auf exogene Einflüsse bietet und wirtschaftlich einsetzbar ist, wird ein solches vorgeschlagen, bei dem für mindestens einen Teil des biologischen Systems die Produktionsrate von mindestens einem Spurengas kontinuierlich oder semi-kontinuierlich bestimmt wird, indem
a. das biologische System einem exogenen Einfluss ausgesetzt wird,
b. die Konzentration mindestens eines Spurengases in der das biologische System umgebenden Gasmenge kontinuierlich oder semi-kontinuierlich mit mindestens einer hochauflösenden Analyseeinheit gemessen wird, und
c. anhand des zeitlichen Verlaufs der Spurengaskonzentration als Maß für den zeitlichen Verlauf der Produktionsrate bestimmt wird, ob bzw. wie das biologische System auf den exogenen Einfluss reagiert.

## Beschreibung

Die Erfindung betrifft ein Verfahren zur Bestimmung der Reaktion mindestens eines biologischen Systems, insbesondere einer Pflanze, auf mindestens einen exogenen Einfluss.

Nutz- und Zierpflanzen werden in der Forschung und in landwirtschaftlichen Betrieben mit den unterschiedlichsten Präparaten, beispielsweise vitalitätssteigernden Präparaten, Düngemitteln oder Pflanzenschutzmitteln, behandelt, um sie beispielsweise gegen exogene Einflüsse wie Pilzbefall zu schützen und ihre Vitalität zu steigern. Die Wirkung vieler Präparate beruht auf einer Veränderung im Stoffwechsel der Pflanze, sei es, dass ein Mangel an bestimmten Wirkstoffen oder deren Vorstufen zugeführt und damit ausgeglichen wird, oder dass eine bestimmte, der Aktivierung der Abwehrkräfte bei einer Schutzimpfung vergleichbare, systemische oder lokale Reaktion der gesamten Pflanze hervorgerufen wird.

Die Wirkung der Präparate wird in der Praxis entweder mit molekularbiologischen Verfahren oder in lange andauernden Versuchsreihen über mehrere Tage und Wochen ermittelt bzw. beobachtet und durch Bonitur optischer Veränderungen und einer Korrelation mit Vitalitätsparametern oder dem Ertrag bewertet. In sogenannten Screening-Verfahren werden eine Vielzahl verschiedener Wirkstoffe in unterschiedlichen Konzentrationen und/oder Kombinationen auf Pflanzen appliziert und die Pflanzenreaktion beurteilt. Auch werden auf mit Präparaten behandelte Pflanzen Pathogene appliziert, um - ebenfalls durch Bonitur - festzustellen, wie die Pflanze auf das Pathogen reagiert.

Neben dem Ansatz, das Wachstum und den Ertrag von Pflanzen durch die Behandlung mit Präparaten zu optimieren, wird auch versucht, Pflanzen zu züchten, die von vorne herein besonders vital und gegen exogene Einflüsse resistent sind. Dabei starten Züchter in der Regel mit bis zu 10.000 (Zucht-)Linien, die sie den verschiedensten Tests unterziehen, um neue Sorten mit verbesserten Eigenschaften zu entwickeln. Die einzelnen Linien werden exogenen Einflüssen ausgesetzt, beispielsweise werden Sporen appliziert, um festzustellen, ob die Pflanze von den Sporen infiziert wird. Auch hierbei ist es üblich, die Pflanze durch Bonitur optischer Veränderungen und einer Korrelation mit Vitalitätsparametern oder dem Ertrag zu bewerten.

Ein wesentlicher Nachteil bei der Bonitur optischer Veränderungen der Pflanze besteht darin, dass es vergleichsweise lange dauert, bis dass sich optische Veränderungen an der Pflanze zeigen. So kann ein optischer Nachweis von Pilzhyphen erst 5 - 7 Tage nach der Applikation von Sporen geführt werden. Gerade bei einer Vielzahl durchzuführender Versuche ist der zeitliche Aufwand daher beträchtlich.

Ein seit langem bekannter und anerkannter Stressindikator bei Pflanzen ist das Ethylen, welches auch die Funktion eines Phytohormons besitzt. Änderungen des Stoffwechsels aufgrund exogener Einflüsse spiegeln sich häufig in einer Veränderung der Ethylenproduktion wieder. In Laborversuchen hat man diese Kenntnis bislang ausgenutzt, um anhand der Menge des innerhalb eines bestimmten Zeitraums von einer Pflanzenprobe produzierten Ethylens Rückschlüsse auf Veränderungen im Stoffwechsel der Pflanze ziehen zu können. Dabei werden für die Analyse Pflanzenproben, beispielsweise Blattscheiben, einzelne Blätter oder Früchte, für mehrere Stunden in kleine, geschlossene Glasgefäße gefüllt, in denen sich das Ethylen akkumulieren kann (Headspace-Technologie). Nach einer Zeit von 1 bis 3 Stunden wird eine Gasprobe aus dem Gefäß entnommen, in einen Gaschromatographen injiziert und gemessen.

Diese Methode hat verschiedene wesentliche Nachteile. Es hat sich gezeigt, dass eine Pflanze auf eine Verletzung beim Abschneiden einer Pflanzenprobe mit Stress reagiert, der sich in einer erhöhten Ethylenproduktion äußert. Soll die Reaktion einer Pflanze auf exogene Einflüsse wie beispielsweise eine Inokulation oder die Applikation eines Wirkstoffs durch eine Bestimmung der von der Pflanzenprobe produzierten Ethylenmenge untersucht werden, werden die Messergebnisse durch die erhöhte Ethylenproduktion als Reaktion auf die Verletzung der Probe verfälscht. Es kann also nur die Reaktion auf eine Mehrzahl von Stressoren bestimmt werden.

Somit kann man zwar mit solchen Messungen feststellen, dass die Pflanze auf äußere Einflüsse reagiert, es besteht jedoch nicht die Möglichkeit zu bestimmen, welches die genaue Ursache für eine erhöhte Ethylenproduktion ist. Insofern ist dieses Verfahren zur Beurteilung der Eigenschaften der untersuchten Pflanze ungeeignet.

Vor diesem Hintergrund liegt der Erfindung die Aufgabe zugrunde, ein Verfahren der eingangs genannten Art zur Verfügung zu stellen, das eine genauere Möglichkeit zur Untersuchung der Reaktion von biologischen Systemen auf exogene Einflüsse bietet und wirtschaftlich einsetzbar ist.

Diese Aufgabe wird gelöst mit einem Verfahren, bei dem für mindestens einen Teil des biologischen Systems die Produktionsrate von mindestens einem Spurengas kontinuierlich oder semi-kontinuierlich bestimmt wird, indem
a. das biologische System einem exogenen Einfluss ausgesetzt wird,
b. die Konzentration mindestens eines Spurengases in der das biologische System umgebenden Gasmenge kontinuierlich oder semi-kontinuierlich mit mindestens einer hochauflösenden Analyseeinheit gemessen wird, und
c. anhand des zeitlichen Verlaufs der Spurengaskonzentration als Maß für den zeitlichen Verlauf der Produktionsrate bestimmt wird, ob bzw. wie das biologische System auf den exogenen Einfluss reagiert.

Unter einem biologischen System wird hier und im Folgenden jegliche Form eines Organismus verstanden, der einen Stoffwechsel hat und bei dem aufgrund von Stoffwechselprozessen Spurengase freigesetzt werden. Unter Spurengasen werden dabei insbesondere solche verstanden, deren Anteil an der Atmosphäre unter Umgebungsdruck weniger als 1 Vol.-%, insbesondere weniger als 0,01 Vol.-% ist. Auch werden unter biologischen Systemen solche aus mehreren Organismen, wie beispielsweise Pflanze/Pathogen, Organ/Krankheitserreger verstanden, ebenso wie die Kombination aus Organismus und Wirkstoff bzw. Wirkstoffkombination.

Bei dem vorliegenden erfindungsgemäßen Verfahren wird ausgenutzt, dass der Stoffwechsel einer Vielzahl biologischer Systeme auf exogene Einflüsse, worunter grundsätzlich jeder äußere Einfluss zu verstehen ist, der die Entwicklung des biologischen Systems beeinflusst, in typischer Weise reagiert. So hat die Spurengasproduktion von biologischen Systemen, beispielsweise die Ethylenproduktion bei einer Pflanze, je nach Art des exogenen Einflusses einen anderen, für diesen Einfluss typischen Verlauf, ähnlich wie der für eine Krankheit typische Verlauf einer Fieberkurve. Soll somit beispielsweise die Resistenz einer mit einem Wirkstoff behandelten Pflanze gegen eine Inokulation mit einem Pathogen überprüft werden, kann mit dem erfindungsgemäßen Verfahren beispielsweise der zeitliche Verlauf der Produktionsrate des Spurengases Ethylen bestimmt und überprüft werden, ob der gemessene zeitliche Verlauf einem Vergleichsmuster des zeitlichen Verlaufs der Ethylenproduktionsrate einer Pflanze entspricht, die unbehandelt dem Pathogen ausgesetzt worden ist und von dem Pathogen infiziert worden ist. Entspricht der gemessene Verlauf dem Vergleichsmuster, ist die Pflanze nicht gegenüber dem Pathogen resistent. Ist der Verlauf anders als das Vergleichsmuster, wurde mit dem Wirkstoff ein Erfolg erzielt. Dabei kommt es weniger auf die Menge des produzierten Ethylens, als vielmehr auf den zeitlichen Verlauf, wann die Produktionsrate ansteigt und wieder absinkt, an.

Voraussetzung hierfür ist, dass die verwendete Analyseeinheit die Spurengase in einer ausreichend hohen Auflösung messen kann. Hier kommen insbesondere Laserspektroskopieverfahren in Betracht, mit denen Spurengase in einer Konzentration im ppm-, ppb- bzw. ppt-Bereich nachweisbar sind.

Auch kommt es bei der Messung der vom biologischen System produzierten Spurengase auf die Zeitauflösung bei der Messung an. Je nach untersuchtem biologischen System sollen die Messungen bei semi-kontinuierlicher Messung der Spurengaskonzentration nicht mehr als 1 Stunde auseinander liegen. Bevorzugt werden bei einer semikontinuierlichen Messung Messfrequenzen von 1/600 Hz, dass entspricht einem Abstand der Messungen von 10 Minuten, oder höher bis zu einer Taktrate von 3 Hz angewandt, wobei sich die höchste Messfrequenz zum einen aus der bei dem gewählten Messaufbau maximal möglichen Messfrequenz und zum anderen aus der Anzahl der parallel zu untersuchenden biologischen Systeme ergibt.

Bei einer zeitlich aufgelösten Messung ist es möglich, einzelne Spitzen in der durch die Messungen bestimmten Produktionsrate des Spurengases einer Ursache zuzuordnen. So geht aus Untersuchungen von Teilen bestimmter Pflanzen, insbesondere aus der Untersuchung von deren Blättern hervor, dass innerhalb der ersten ein bis drei Stunden, nachdem das Blatt von der Pflanze abgetrennt und mit einem Pathogen behandelt worden ist, ein erster Anstieg der Ethylenproduktionsrate mitunter ausschließlich auf den durch das Abschneiden des Blattes verursachten Wundstress zurückzuführen ist. Die Stoffwechselreaktion des Blattes auf das Pathogen erfolgt dann erst zu einem späteren Zeitpunkt.

Insofern besteht gegenüber den aus dem Stand der Technik bekannten Verfahren der Vorteil, dass mit einer nicht-invasiven Untersuchungsmethode unmittelbar festgestellt werden kann, wann das biologische System auf einen exogenen Einfluss reagiert, und gleichzeitig bei Betrachtung des zeitlichen Verlaufs der Produktionsrate eines Spurengases festgestellt werden kann, ob die Reaktion des biologischen Systems auf den exogenen Einfluss zurückzuführen ist. Somit können die Stoffwechselprozesse an ein und demselben Organismus über die gesamte Dauer eines Experimentes detailliert ausgewertet werden.

Aufgrund der Tatsache, dass mit dem erfindungsgemäßen Verfahren die Stoffwechselreaktionen mit einer nicht-invasiven Methode beobachtet und beurteilt werden, können die Schlussfolgerungen in bezug auf die Eigenschaften des untersuchten Organismus wesentlich früher gezogen werden, als es bislang bei der Boniturprüfung makroskopischer Veränderungen, die erst zu einem wesentlich späteren Zeitpunkt auftreten, möglich ist.

Dieser Vorteil ist umso größer als mit der kontinuierlichen oder semi-kontinuierlichen Messung der Konzentration von Spurengasen neben dem zeitlichen Verlauf der Produktionsrate auch die Menge des während der Messdauer produzierten Spurengases bestimmt werden kann. Somit ist es möglich, neben einer qualitativen Aussage darüber, dass eine Reaktion des untersuchten biologischen Systems auf den exogenen Einfluss vorliegt, auch eine quantitative Aussage über die Art, wie das biologische System auf den exogenen Einfluss reagiert, zu treffen.

Als biologische Systeme, die mit dem erfindungsgemäßen Verfahren bevorzugt untersucht werden können, kommen insbesondere Pflanzen einschließlich transgene Pflanzen oder Teile davon, insbesondere Blätter, Blattscheiben oder Früchte sowie Zellsuspensionen oder Suspensionen mit Protoplasten in Betracht. Bei der Untersuchung von Pflanzen oder deren Teilen ist insbesondere Ethylen ein guter Stressindikator für externe Einflüsse. Mit der Bestimmung der Produktionsraten von Ethan, Pentan oder Ethylen können Rückschlüsse auf oxidative Prozesse innerhalb des Organismus gezogen werden, die irreversible Schädigungen hervorrufen. Die Beteiligung von Stickstoffmonoxid an der Resistenz von Pflanzen gegen Pathogenbefall wird wissenschaftlich diskutiert. Eine vergleichbare Schlüsselrolle wie beim Menschen ist zu erwarten. Das Stickstoffmonoxid kann demnach ein Resistenzindikator für Pflanzen sein.

Das Verfahren ist aber ebenso bevorzugt verwendbar für tierische oder menschliche Organismen, deren Organe, Gewebe, Blut, Blutplasma oder auch für Zellkulturen. So kann insbesondere aus dem Verlauf der NO-Produktionsrate, die in der Atemluft eines Menschen nachgewiesen werden kann, ein Rückschluss auf endogene Prozesse gewonnen werden. Wird beispielsweise einem Menschen ein ¹⁵NO-dotierter Wirkstoff verabreicht, kann über die Bestimmung der Konzentration von ¹⁵NO geschlossen werden, wie gut und in welchem Zeitraum der Wirkstoff vom Menschen resorbiert worden ist. Ein solcher - ebenso stoffwechselbasierter - Nachweis der Reaktion eines Organismus auf einen Wirkstoff war bislang nicht möglich.

Des weiteren kann man auch bei tierischen oder menschlichen Organismen Ethan, Pentan und Ethylen zur Beurteilung von oxidativen Prozessen in biologischem Gewebe aufgrund einer Schädigung durch Radikale nachweisen.

Als exogene Einflüsse, deren Wechselwirkung mit einem Organismus besonders bevorzugt untersucht werden können, sind die Applikation von Krankheitserregern bzw. Pathogenen und Wirkstoffe sowie Wirkstoffkombinationen zu nennen. Insbesondere kann die Verträglichkeit von Organismen und Wirkstoffen untersucht werden, ebenso wie die Wirksamkeit von Wirkstoffen, bevor oder nachdem auf einen Organismus ein Krankheitserreger oder ein Pathogen appliziert worden ist. Weitere exogene Einflüsse wie Temperatur, Sauerstoff/Kohlendioxidkonzentration in der den Organismus umgebenden Atmosphäre, PH-Wert in der zugeführten Nahrung o.ä. und ihr Einfluss auf den Stoffwechsel des Organismus können ebenso zeitnah und ohne direkten Eingriff in den Organismus untersucht werden.

Aufgrund der Tatsache, dass durch zeitlich aufgelöste Referenzmessungen festgestellt werden kann, in welchem typischen Zeitraum ein biologisches System nach der Applikation eines exogenen Einflusses auf einen exogenen Einfluss reagiert, kann das biologische System beispielsweise durch genetische Veränderung oder durch eine Applikation von Wirkstoffen variiert werden, und untersucht werden, ob die Reaktion auf den gleichen, nachfolgenden exogenen Einfluss immer noch und im gleichen Maße auftritt. Hierfür kann es ausreichen, dass lediglich während eines für die Reaktion typischen Zeitraums die Konzentrationsmessung durchgeführt wird. Somit kann die Messdauer wesentlich verkürzt werden, was gerade bei Reihenuntersuchungen zu einer weiteren erheblichen Verkürzung der für eine Versuchsreihe benötigten Zeit resultiert. Im einfachsten Fall ist es möglich, aus der Tatsache, daß in einem typischen Zeitraum kein Anstieg der maßgeblichen Spurengaskonzentration zu messen ist, zu schließen, dass das untersuchte biologische System gegenüber dem applizierten exogenen Einfluss resistent ist.

Als hochauflösende Analyseeinheit eignen sich insbesondere photoakustische Sensoren, Faraday-Rotations-Spektrometer, Cavity-Leak-Out-Spektrometer bzw. Cavity-Ring-Down-Spektrometer und/oder Massenspektrometer.

Wird ein Faraday-Rotations-Spektrometer verwendet, ist es von Vorteil, wenn das biologische System bei Normaldruck, die Messung im Spektrometer aber bei verringertem Druck erfolgt, wobei der Absolutdruck weniger als 30 mbar, insbesondere 25 mbar betragen sollte. Hiermit wird erreicht, dass selbst kleine zu untersuchende Gasproben ein vergleichsweise großes Volumen einnehmen und ohne eine wesentliche Verdünnung der Gasprobe durch ein Trägergas ausgemessen werden können. Auch Cavity-Leak-Out-Spektrometer bzw. Cavity-Ring-Down-Spektrometer werden bevorzugt mit Unterdruck betrieben. Voraussetzung für das Durchführen der Messungen bei Unterdruck ist, dass sich die zu untersuchenden biologischen Systeme in einem geschlossenen Behälter befinden, der mit dem Spektrometer über eine Leitung verbunden ist.

Aufgrund der Tatsache, dass man mit der nicht-invasiven Untersuchung von Stoffwechselprozessen viel schneller als bislang ein Untersuchungsergebnis erhält, kann das Verfahren besonders vorteilhaft für breite Versuchsreihen zur Bestimmung der Reaktion einer Vielzahl von biologischen Systemen auf exogene Einflüsse verwendet werden, wobei für jedes biologische System die Produktionsrate von mindestens einem Spurengas semi-kontinuierlich bestimmt wird, und wobei
a. nacheinander von jeder der die biologischen Systeme umgebenden Gasmengen eine Gasprobe über mindestens einen Gasmultiplexer mindestens einer hochauflösenden Analyseeinheit zugeführt wird,
b. die Konzentration eines oder mehrerer Spurengase in der jeweiligen Gasmenge gemessen und
c. in einer Auswerteeinheit dem jeweiligen biologischen System zugeordnet wird.

Dabei ist es nicht unbedingt notwendig, dass sich jedes der zu untersuchenden biologischen Systeme in einem abgeschlossenen Behälter befindet. Da es mehr auf das Muster des zeitlichen Verlaufs der Produktionsrate und weniger auf die absolute Menge der produzierten Spurengase ankommt, kann es ausreichen, wenn der zeitliche Verlauf der Konzentration eines Spurengases in einer nicht abgeschlossenen Umgebung des biologischen Systems bestimmt wird. Beispielsweise kann der zeitliche Verlauf der lokalen Spurengaskonzentration an verschiedenen Pflanzen in einem Gewächshaus bestimmt werden. Voraussetzung hierfür ist, dass sich lokal unterschiedliche Gaszusammensetzungen ausbilden können, was bedeutet, dass die Luft im Gewächshaus im wesentlichen frei von Strömungen ist. Auch sollte der zeitliche Abstand lokaler Messungen voneinander vergleichsweise gering sein, so dass man eine genaue Kurve des Konzentrationsverlaufs erhält und auf diese Weise mögliche Störungen, beispielsweise durch Messpersonal verursachte Luftbewegungen, als solche erkennen kann.

Das Sammeln der einzelnen Gasproben und das Zuführen der einzelnen Gasproben zur Analyseeinheit kann beispielsweise über ein als Gasmultiplexer fungierendes Leitungssystem erfolgen, das eine Vielzahl von Probenentnahmeleitungen aufweist, die zu den einzelnen biologischen Systemen führen, über steuerbare Ventile mit einer Zufuhrleitung für die Analyseeinheit verbunden sind, und das ebenso über ein steuerbares Ventil mit einer Spülbzw. Trägergasversorgung verbunden ist, wobei im Wechsel an einem biologischen System eine Gasprobe angesaugt und der Analyseeinheit zugeführt wird, danach die Zuführleitung und gegebenenfalls die Probenentnahmeleitung mit dem Spülgas bzw. Trägergas gespült wird, und danach eine nächste Gasprobe von einem anderen biologischen System genommen wird, usw.

Um ein Vermischen der lokalen Gasvolumina benachbarter biologischer Systeme zu verhindern, wird das Gas bevorzugt in einem Gasraum gesammelt, wobei der Gasraum vorzugsweise durch einen geschlossenen Behälter oder eine nah über dem biologischen System angeordnete Haube gebildet werden kann. Befindet sich das biologische System in einem geschlossenen Behälter, muss dafür Sorge getragen werden, dass die Gasmenge, die als Gasprobe dem Gasraum entnommen wird, durch ein Spül- bzw. Trägergas ersetzt wird. Dieses Gas kann Luft sein, sofern eine gleichbleibende Luftzusammensetzung, mindestens im Hinblick auf die Konzentration der zu messenden Spurengase, während einer Versuchsreihe garantiert werden kann, damit die Messungen nicht durch den Einfluss unterschiedlicher Spül- bzw. Trägergaszusammensetzungen verfälscht werden.

Insbesondere für groß angelegte Reihenversuche an kleinvolumigen biologischen Systemen, insbesondere Blattscheiben, Zellsuspensionen, Blut, Blutplasma oder Zellkulturen eignen sich als Gasräume die Wells von Mikrotiterplatten. Da die Wells in der Regel bei einer typische Standardgröße mit einem Durchmesser von 6,5 mm und einer Höhe von 10 - 20 mm vergleichsweise klein sind, werden nur geringe Stoffmengen für die Untersuchung benötigt, und zwar sowohl in Hinsicht auf das biologische System, als auch im Hinblick auf die für den exogenen Einfluss benötigten Wirkstoffe bzw. Krankheitserreger oder Pathogene. Dementsprechend ist es grundsätzlich von Vorteil, wenn der das biologische System umgebende bzw. daran angrenzende Gasraum so klein wie möglich ist.

Im weiteren ist es vorteilhaft, wenn die Umgebungsbedingen im Gasraum im Hinblick auf die zu führende Messung hinsichtlich Beleuchtung und klimatischen Bedingungen wie insbesondere Feuchte und Temperatur für das biologische System bzw. für die Versuchsaufgabe optimiert sind. So kann in ein das zu untersuchende biologische System aufnehmendes Gefäß eine Agarschicht als Feuchtepuffer verwendet werden, damit beispielsweise Blattscheiben für einen längeren Zeitraum für eine Dauermessung verwendbar sind. Ebenso sollten Temperatur und Belichtung des biologischen Systems, ebenso wie die Sauerstoff- und Kohlendioxidkonzentration im Gefäß einstellbar sein.

Wie bereits erwähnt eignet sich das Messverfahren besonders gut, um verschiedene Zuchtlinien oder gentechnische Variationen einer Pflanze zu untersuchen und über die Produktionsrate der gemessenen Spurengase zu bestimmen, ob eine jeweilige Zuchtlinie gegen den untersuchten exogenen Einfluss resistent ist. Darüber hinaus ist das Verfahren besonders geeignet, um beispielsweise bei der Entwicklung von Herbiziden festzustellen, ab welcher Dosierung ein Wirkstoff oder eine Wirkstoffkombination eine zu bekämpfende Pflanze wirksam vernichtet, in dem die Stressreaktion solcher Pflanzen auf die Applikation des Herbizids untersucht wird. Auch kann über entsprechende Messreihen festgestellt werden, ab welcher Dosierung eine weitere Zunahme der Dosierung wirkungslos ist, und zwar dann, wenn festgestellt wird, dass die Stressreaktion der Pflanze sich weder in ihrem zeitlichen Verlauf, noch in ihrer Intensität mehr wesentlich verändert.

Schließlich ist das Verfahren besonders gut geeignet für eine auf Dauer angelegte Untersuchung von Pflanzen, die zunächst mit einem Wirkstoff oder einer Wirkstoffkombination behandelt worden sind und auf die danach ein Pathogen appliziert wird oder umgekehrt, bei welchem Wirkstoff bzw. welcher Wirkstoffkombination und/oder in welcher Dosierung ein optimales Ergebnis hinsichtlich der Wirkung des Wirkstoffs in bezug auf die Unterstützung der Pflanze gegen das Pathogen und in bezug auf die Stresswirkung des Wirkstoffs bzw. der Wirkstoffkombination auf die Pflanze erzielt werden kann.

Ebenso ist das Verfahren besonders gut geeignet, um verschiedene Dosierungen und Kombinationen einzelner Wirkstoffe und die Verträglichkeit des untersuchten Organismus hierauf zu untersuchen. Auch kann bei solchen Versuchen die Eignung des Wirkstoffs überprüft werden, die Inokulation bzw. Infektion von Organismen mit Pathogenen bzw. Krankheitserregern zu verhindern oder bereits infizierte bzw. inokulierte Organismen bei einem Heilungsprozess zu unterstützen.

Insbesondere bei kleinen zu untersuchenden biologischen Systemen wie Blattscheiben, Zellsuspensionen oder Protoplasten sowie Zellkulturen eignen sich Mikrotiterplatten mit einer Vielzahl von Wells für Reihenuntersuchungen. Sie haben den Vorteil, dass das Volumen der Wells, in denen die biologischen Systeme eingebracht und die als Gassammelraum dienen, vergleichsweise klein sind, so dass die von den biologischen Systemen produzierten Spurengase im zur Verfügung stehenden Gasraum vergleichsweise wenig verdünnt werden. Um zu verhindern, dass die zu messenden Spurengase aus einem Well entweichen, ist es von Vorteil, wenn die Oberseite der Wells mit einem Septum verschlossen ist. Auf diese Weise kann regelmäßig mit einer Kanüle eine Gasprobe aus dem Well entnommen werden, ohne dass vor und oder nach der Gasprobenentnahme die Gasmenge im Well durch äußere Einflüsse verändert wird.

In der Regel ist ein Septum lichtundurchlässig. Um insbesondere bei zu untersuchenden Blattscheiben den Stoffwechselprozess nicht dadurch zu stören, dass die Blattscheibe keiner Lichteinstrahlung mehr ausgesetzt sind, ist es von Vorteil, wenn die Wells eine Gehäusewandung aufweisen, die lichtdurchlässig ist.

Einzelne Aspekte der Erfindung werden im Folgenden anhand von einer eine bevorzugte, für das erfindungsgemäße Verfahren modifizierte Mikrotiterplatte zeigenden Figur sowie anhand von beispielhaften Messkurvengraphiken näher erläutert.

Es zeigen
- Figuren 1a - 1d: eine für das erfindungsgemäße Verfahren modifizierte Mikrotiterplatte,
- Abbildung 1: den Verlauf der Ethylenkonzentration einer Buschbohnenpflanze nach einer Paraquat-Applikation,
- Abbildung 2: den Verlauf der Ethylenemission von Weizenpflanzen infolge eines Pathogenbefalls mit Braun- und Gelbrost,
- Abbildung 3: den Verlauf der Ethylenemission von Weizenpflanzen infolge eines Pathogenbefalls mit Mehltau,
- Abbildung 4: den Beginn des Verlauf der Ethylenemission eines Gurkenblattes infolge von Mehltaubefall,
- Abbildung 5: den vollständigen Verlauf der in Abbildung 4 dargestellten Ethylen-emission des mit Mehltau infizierten Gurkenblattes,
- Abbildung 6: den Verlauf der kumulierten Ethylenkonzentration als Stammfunktion des Verlauf des in Abbildung 5 dargestellten Ethylenkonzentration,
- Abbildung 7: der sigmoide Kurvenverlauf des in Abbildung 6 dargestellten Verlaufs der über die Zeit kumulierten Ethylenkonzentration, und
- Abbildung 8: die Abweichungen des Verlaufs der in Abbildung 7 dargestellten kumulierten Ethylenkonzentration von dem in Abbildung 7 dargestellten sigmoiden Verlauf.

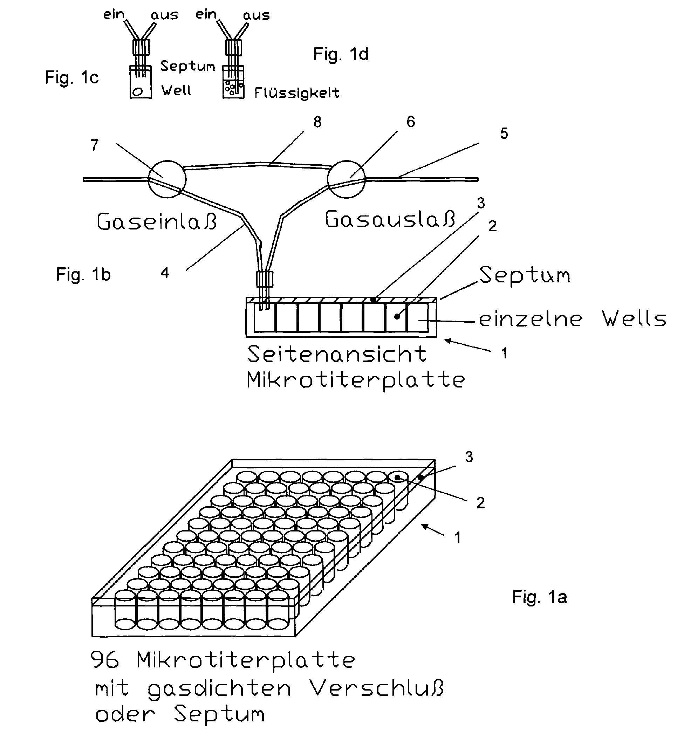

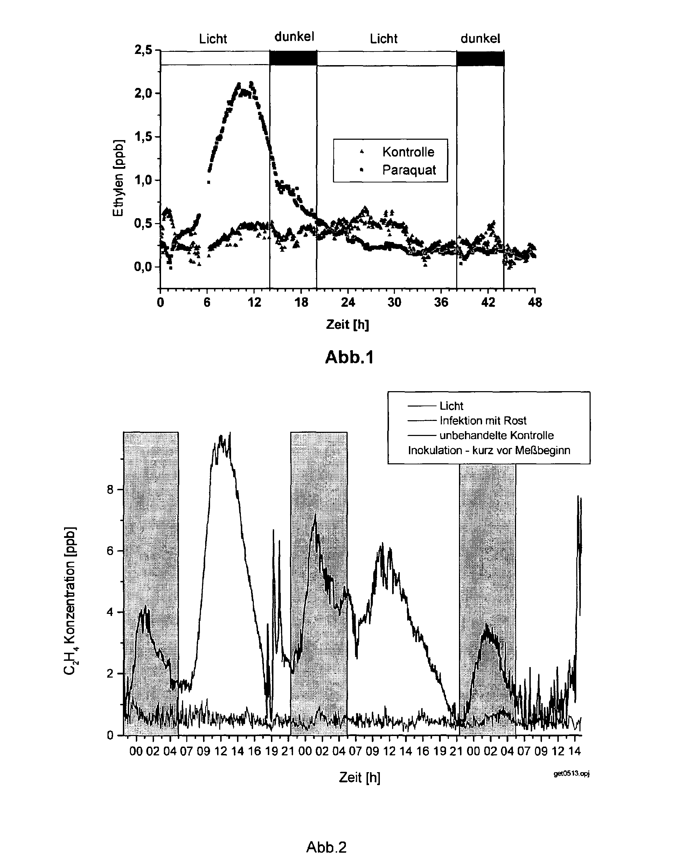

Figur 1a zeigt eine isometrische Ansicht einer Mikrotiterplatte **1** mit einer Vielzahl von 96 in Reihen und Spalten angeordneten Wells **2**. Die Öffnungen der Wells **2** sind mit einem sich über die gesamte Oberseite der Mikrotiterplatte **1** erstreckendes Septum **3** verschlossen.

Die Mikrotiterplatte **3** ist in Figur 1b in Seitenansicht dargestellt. Eine Gaseinlasskanüle **4** und eine Gasauslasskanüle **5** sind durch das Septum **3** in eines der Wells **2** eingeführt, wobei bei eine Gasprobe bei der Entnahme über die Gasauslasskanüle **5** abgeführt und einer nicht dargestellten Analyseeinheit zugeführt wird, und über die Gaseinlasskanüle **4** ein Spül- bzw. Trägergas nachgeführt werden kann. Die Kanülen **4**, **5** können nach Entnahme der Gasprobe aus dem Well **2** herausgezogen werden, wonach sich das Septum **3** automatisch wieder gasdicht verschließt. Hierdurch ist es möglich, aus einem verschlossenen Well **2** eine Gasprobe zu entnehmen, ohne dass die Gasprobe durch Umgebungsluft oder die Gasmengen in den benachbarten Wells **2** verunreinigt werden kann.

Gaseinlasskanüle **4** und Gasauslasskanüle **5** sind über Ventile **6**, **7** mit einer Bypass-Leitung verbunden **8**, so dass ein Spül- oder Trägergas an der Gaseinlasskanüle **4** und der Gasauslasskanüle **5** vorbeigeleitet werden kann. So kann beispielsweise bei entsprechender Ventilsteuerung ein sich in Reihe anschließendes Kanülenpaar, dass in ein benachbartes Well eingesteckt ist (hier nicht dargestellt) für den Gaseinlass und den Gasauslass mit einem Spül- oder Trägergas zur Probenentnahme versorgt werden. Auf diese Wiese wird es möglich, eine Vielzahl von Wells übe eine einzige Leitung, von der Gaseinlass- und Gasauslasskanülenpaare in die Wells abzweigen, mit der Analyseeinheit zu verbinden.

Sofern sichergestellt ist, dass die Gasauslasskanüle **5** nach jeder Gasprobenentnahme mit einem Spülgas gespült worden ist, kann es ausreichen, dass lediglich eine Gaseinlasskanüle **4** und eine Gasauslasskanüle **5** samt zugehörigem Leitungssystem vorgesehen sind, wobei dann die Kanülen **4**, **5** über eine Robotik von der Probenentnahme bei einem Well **2** nach dem Spülen der Gasauslasskanüle **5** zur Probenentnahme in ein anderes Well **2** eingebracht wird.

Für eine Reihenuntersuchung ist es aber mindestens ebenso sinnvoll, wenn jedes der Wells **2** über eigene Leitungen zum Gaseinlass und Gasauslass dauerhaft mit einem Gasmultiplexer verbunden ist, der dann automatisch Gasproben von jedem der Wells nacheinander der Analyseeinheit zuführt.

Figuren 1c und 1d zeigen zwei unterschiedliche Anordnungen für die Kanülen **4**, **5** im Well **2**. Bei einer festen Probe **9** liegen die Enden beider Kanülen **4**, **5** im oberen Bereich des Wells **2**. Werden Flüssigkeiten **10** wie beispielsweise Zellsuspensionen oder andere flüssige Proben untersucht, reicht das Ende der Gaseinlasskanüle **4** bis in die Suspension, so dass die Probe mit einem Trägergas gespült werden kann, das dann über diffusiven Gasaustausch Spurengase aufnimmt. Die somit erzeugte Gasprobe wird dann über die Gasauslasskanüle **5**, deren Ende oberhalb des Flüssigkeitsspiegels angeordnet ist, abgeführt.

Abbildung 1 zeigt eine Messkurve der Ethylenemission einer einzelnen Buschbohnen-Pflanze (*Phaseolus vulgaris* L. var. nanus L. cv. Rocdor) nach einer Paraquat-Applikation von 20 Tropfen (V= 0,5 µl) und einer Konzentration von 0,3 mmol/l mit einer Mikrotiterspritze auf die Primärblätter der Pflanze. Ca. 3 Stunden nach der Applikation ist ein deutlicher Anstieg der Ethylenkonzentration zu erkennen, der von einem niedrigen Niveau bei ca. 0,5 ppb beginnt und nach ca. 11 Stunden nach der Applikation ein Maximum von ca. 2,0 ppb erreicht. Danach sinkt die Ethylenkonzentration wieder, wobei auffällt, dass der Gradient der Konzentrationsabnahme während einer Dunkelphase im Zeitraum von 14 bis 20 Stunden nach der Applikation deutlich geringer ist als vor der Dunkelphase. Nach 24 Stunden liegt die Ethylenkonzentration wieder vergleichsweise konstant bei einer Größenordnung von 0,3-0,4 ppb. Die Vergleichsmessung einer unbehandelten Buschbohnen-Pflanze zeigt eine vergleichsweise konstante Ethylenkonzentration im Bereich von 0,3 bis 0,5 ppb.

Abbildung 2 zeigt die Ethylenemission von Weizenpflanzen infolge eines Pathogenbefalls mit Braun- und Gelbrost nach einer Inokulation der Weizenpflanzen mit einer Sporenlösung unmittelbar vor Messbeginn (Zeitpunkt 0 h). Ein erstes Ethylenmaximum ist während einer Dunkelphase bereits nach 2 Stunden bei ca. 4 ppb zu erkennen. Weitere Maxima folgen, wobei insbesondere in der Tageslichtphase 12 Stunden nach der Inokulation ein absolutes Maximum von ca. 10 ppb erreicht ist. Die Parallelmessung zu einer unbehandelten Kontrollpflanze zeigt eine vergleichsweise konstante Ethylenkonzentration von zwischen 0,5 und 1,0 ppb.

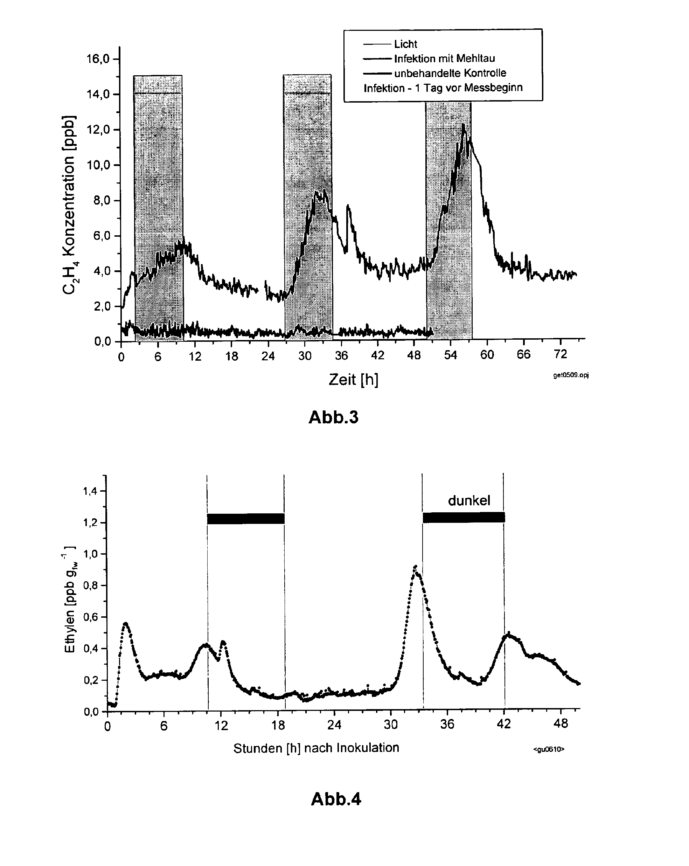

Abbildung 3 zeigt die Ethylenemission von Weizenpflanzen infolge eines Pathogenbefalls mit Mehltau. Die Inokulation der Weizenpflanzen mit Pilzsporen erfolgte 24 Stunden vor dem Beginn der Messung. Der Konzentrationsverlauf zeigt einen charakteristischen Anstieg jeweils zum Ende der Dunkelphasen, wobei die maximale Ethylenkonzentration mit jeder weiteren Dunkelphase steigt und ein absolutes Maximum von ca. 12 ppb 54 Stunden nach Messbeginn gemessen wird, während die Ethylenkonzentration in jeder Lichtphase bis zu ihrem Ende hin absinkt. Eine Vergleichsmessung mit unbehandelten Kontrollpflanzen zeigt ebenso wie in Abbildung 2 eine vergleichsweise konstante Ethylenkonzentration von ca. 0,5 bis 1,0 ppb.

Der Vergleich der Ethylenkonzentrationskurven von den mit Braun- und Gelbrost inokulierten Weizenpflanzen zu den mit Mehltau inokulierten Weizenpflanzen ist deutlich voneinander unterscheidbar, so dass sich aus dem Verlauf der Kurven klare Rückschlüsse auf die Ursache der Ethylenproduktion der Pflanzen schließen lassen.

Abbildung 4 zeigt den Anfang des Verlaufs der Ethylenemission eines Gurkenblattes infolge von Mehltaubefall. Beobachtet werden kann eine Primärinfektion bei dem zweiten relativen Konzentrationsmaximum nach ca. 10 h, nachdem ein erstes relatives Konzentrationsmaximum nach ca. 2 Stunden aufgrund des Wundstresses (Wundethylens) abgeklungen ist. Ab der zweiten Dunkelphase bildet sich ein Doppelpeak kurz vor Eintritt der Dunkelheit bzw. vor Helligkeit aus. Obwohl die gemessenen Ethylenkonzentrationen in einem Bereich von ca. 0,1 bis 0,9 ppb liegen, lässt sich der Kurvenverlauf eindeutig erkennen. Hieraus lässt sich ableiten, dass der Signal-/Rauschabstand bei der verwendeten Analyseeinheit, hier ein photoakustischer Sensor, trotz äußerst geringer Konzentrationen ausreichend hoch ist.

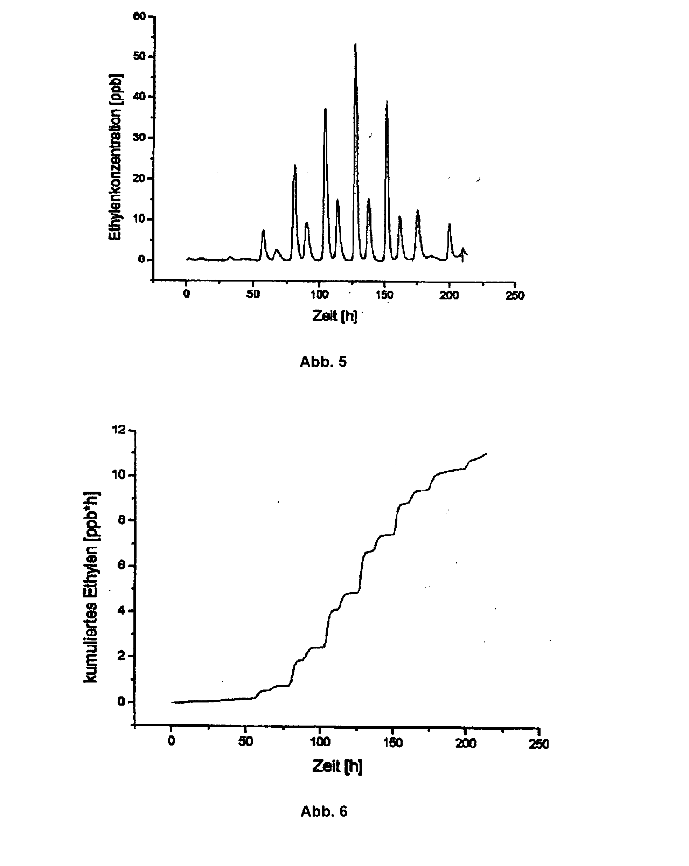

In Abbildung 5 ist der gesamte zeitliche Verlauf der Ethylenemission in der Umgebung eines mit Mehltau infizierten Gurkenblattes dargestellt. Es fällt auf, dass gut 125 Stunden nach der Inokulation mit Mehltau die Produktionsrate des Gurkenblattes ein absolutes Maximum erreicht. Aus einem Vergleich der Abbildungen 4 und 5 ergibt sich aber, dass sich eine Reaktion auf den Mehltaubefall schon zu einem sehr frühen Zeitpunkt feststellen lässt, da eine charakteristische Abfolge der relativen Maxima des Verlaufs der gemessenen Ethylenkonzentrationen bereits zu einem sehr frühen Zeitpunkt, nämlich bereits nach 10 Stunden, festgestellt werden.

Um eine quantitative Aussage über die Reaktion des Gurkenblattes zu erhalten, wird aus dem Verlauf der Ethylenkonzentration im Sinne einer Stammfunktion der Verlauf der kumulierten Ethylenkonzentration berechnet.

Der Verlauf der kumulierten Ethylenkonzentration, der mit dem Verlauf der Ethylenkonzentration aus Abbildung 5 korreliert, ist in Abbildung 6 dargestellt. Dieser Kurvenverlauf weist ein regelmäßiges, treppenartiges Muster auf, das ebenso für den Mehltaubefall von Gurkenblättern charakteristisch ist. Aus dem Verlauf der kumulierten Ethylenkonzentration lässt sich ein sogenannter logistischer Kurvenverlauf ermitteln. Dieser logistische Kurvenverlauf wird durch eine geglättete Kurve dargestellt, die einen sigmoiden Verlauf bzw. einen hierzu ähnlichen Verlauf aufweist. Über dieses "Anfitten" des Verlaufs der kumulierten Ethylenkonzentration gemäss Abbildung 6 werden Parameter entwickelt, die einen systemdynamischen Vergleich mit anderen Kurvenverläufen ermöglichen. Mit den auf diese Weise ermittelten Parametern ist eine ganzheitliche Beschreibung des untersuchten biologischen Prozesses möglich, wobei in einem damit charakterisierten biologischen System zu einem sehr frühen Zeitpunkt Prognosen für den weiteren Verlauf des im biologischen System ablaufenden Prozesses erstellt werden können.

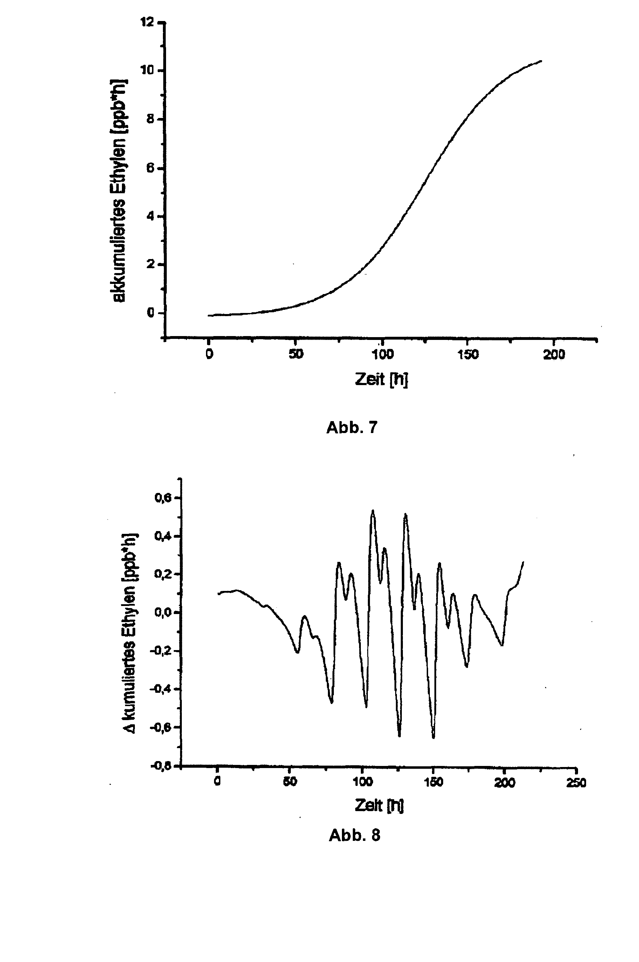

Schließlich ist in Abbildung 8 die verbleibende Differenz zwischen dem in Abbildung 7 dargestellten logistischen Kurvenverlauf und dem in Abbildung 6 dargestellten Verlauf der kumulierten Ethylenkonzentration dargestellt. Auch hieraus lassen sich weitere Informationen für das beobachtete biologische System entnehmen.

Eine derartige mathematische Auswertung der gemessenen Ethylenkonzentrationen wird insbesondere dadurch ermöglicht, dass Störeinflüsse, die auf das biologische System wirken, in dem beobachteten Gasraum, also der Umgebung des biologischen Systems, kontrolliert oder zumindest klar definiert werden können.

Alle der in den Abbildungen dargestellten Messungen wurden in einer vergleichsweise hohen zeitlichen Auflösung mit einer Vielzahl von Messungen pro Stunde durchgeführt, so dass im Ergebnis ein genauer Verlauf der Ethylenproduktionsrate der Pflanzen ablesbar ist. Erst über die Kenntnis des Verlaufs der Produktionsrate lässt sich ein vergleichsweise eindeutiger Rückschluss auf die Ursache der Erhöhung der Produktionsrate ziehen.

Die den Abbildungen 1 bis 8 zugrunde liegenden Messungen wurden dabei so durchgeführt, dass die Umgebung des biologischen Systems ständig mit Frischluft gespült wurde, die über einen Katalysator von Kohlenwasserstoffen zumindest soweit befreit worden ist, dass ein möglicherweise verbliebener Kohlenwasserstoffgehalt unter der Nachweisgrenze des verwendeten Messgerät, hier ein photoakustischer Sensor, lag.

## Patentansprüche

1. Verfahren zur Bestimmung der Reaktion mindestens eines biologischen Systems auf mindestens einen exogenen Einfluss, bei dem für mindestens einen Teil des biologischen Systems die Produktionsrate von mindestens einem Spurengas kontinuierlich oder semi-kontinuierlich bestimmt wird, indem
a. das biologische System einem exogenen Einfluss ausgesetzt wird,
b. die Konzentration mindestens eines Spurengases in der das biologische System umgebenden Gasmenge kontinuierlich oder semi-kontinuierlich mit mindestens einer hochauflösenden Analyseeinheit gemessen wird, und
c. anhand des zeitlichen Verlaufs der Spurengaskonzentration als Maß für den zeitlichen Verlauf der Produktionsrate bestimmt wird, ob bzw. wie das biologische System auf den exogenen Einfluss reagiert.

2. Verfahren nach Anspruch 1, **dadurch gekennzeichnet, dass** die Menge des vom biologischen Systems während der kontinuierlichen oder semi-kontinuierlichen Messung produzierten Spurengases bestimmt wird.

3. Verfahren nach Anspruch 1 oder 2, **dadurch gekennzeichnet, dass** das biologische System eine Pflanze oder ein Teil davon, insbesondere ein Blatt, eine Blattscheibe, eine Frucht, eine Suspension pflanzlicher Zellen oder eine Suspension mit Protoplasten ist.

4. Verfahren nach Anspruch 1 oder 2, **dadurch gekennzeichnet, dass** das biologische System ein tierischer oder menschlicher Organismus, Organ, Gewebe, Blut, Blutplasma oder eine Kultur tierischer oder menschlicher Zellen ist.

5. Verfahren nach einem der Ansprüche 1 bis 4, **dadurch gekennzeichnet, dass** der zeitliche Verlauf der Ethylenkonzentration gemessen wird.

6. Verfahren nach einem der Ansprüche 1 bis 5, **dadurch gekennzeichnet, dass** der zeitliche Verlauf der Konzentration(en) von Pentan, Ethan, Stickstoffoxid und/oder von einem oder mehreren der Isotopomere von Stickstoffoxid gemessen wird.

7. Verfahren nach einem der Ansprüche 1 bis 6, **dadurch gekennzeichnet, dass** der exogene Einfluss mit mindestens einem Pathogen oder Krankheitserreger erzeugt wird.

8. Verfahren nach einem der Ansprüche 1 bis 7, **dadurch gekennzeichnet, dass** der exogene Einfluss mit mindestens einem Wirkstoff oder einer Wirkstoffkombination erzeugt wird.

9. Verfahren nach einem der Ansprüche 1 bis 8, **dadurch gekennzeichnet, dass** insbesondere während eines für eine Reaktion des biologischen Systems auf den exogenen Einfluss typischen Zeitraums die Veränderung der Konzentration mindestens eines Spurengases kontinuierlich oder semikontinuierlich gemessen wird.

10. Verfahren nach einem der Ansprüche 1 bis 9, **dadurch gekennzeichnet, dass** ein photoakustischer Sensor verwendet wird.

11. Verfahren nach einem der Ansprüche 1 bis 10, **dadurch gekennzeichnet, dass** ein Faraday-Rotations-Spektrometer verwendet wird.

12. Verfahren nach Anspruch 11, **dadurch gekennzeichnet, dass** der Druck innerhalb einer Leitung, über die eine Gasprobe von der Umgebung des biologischen Systems zur Analyseeinheit geführt wird, und in der Analyseeinheit selbst gegenüber dem Umgebungsdruck verringert ist und insbesondere weniger als 30 mbar beträgt.

13. Verfahren nach einem der Ansprüche 1 bis 12, **dadurch gekennzeichnet, dass** ein Cavity-Leak-Out-Spektrometer, ein Cavity-Ring-Down-Spektrometer oder ein Massenspektrometer verwendet wird.

14. Verfahren nach einem der Ansprüche 1 bis 13, **dadurch gekennzeichnet, dass** zur Bestimmung der Reaktion einer Vielzahl von biologischen Systemen auf exogene Einflüsse für jedes biologische System die Produktionsrate von mindestens einem Spurengas semi-kontinuierlich bestimmt wird, wobei
a. nacheinander von jeder der die biologischen Systeme umgebenden Gasmengen eine Gasprobe über mindestens einen Gasmultiplexer mindestens einer hochauflösenden Analyseeinheit zugeführt wird,
b. die Konzentration eines oder mehrerer Spurengase in der jeweiligen Gasmenge gemessen und
c. in einer Auswerteeinheit dem jeweiligen biologischen System zugeordnet wird.

15. Verfahren nach Anspruch 14, **dadurch gekennzeichnet, dass** jeweils das von einem der biologischen Systeme produzierte Spurengas in einem Gasraum gesammelt wird,

16. Verfahren nach Anspruch 15, **dadurch gekennzeichnet, dass** jedes biologische System in einem Well einer Mikrotiterplatte enthalten ist.

17. Verfahren nach Anspruch 15 oder 16, **dadurch gekennzeichnet, dass** die Umgebungsbedingen im Gasraum im Hinblick auf die zu führende Messung hinsichtlich Beleuchtung und klimatischen Bedingungen wie insbesondere Feuchte und Temperatur für das biologische System optimiert sind.

18. Verfahren nach einem der Ansprüche 15 bis 17, **dadurch gekennzeichnet, dass** der das biologische System umgebende oder daran angrenzende Gasraum so klein wie möglich ist.

19. Verfahren nach einem der Ansprüche 14 bis 18, **dadurch gekennzeichnet, dass** die biologischen Systeme verschiedene Zuchtlinien oder gentechnische Variationen einer Sorte sind, und über die Produktionsrate der Spurengase bestimmt wird, ob eine jeweilige Zuchtlinie gegen den exogenen Einfluss resistent ist.

20. Verfahren nach einem der Ansprüche 14 bis 19, **dadurch gekennzeichnet, dass** mindestens einige der biologischen Systeme sich nicht wesentlich voneinander unterscheiden, aber mit unterschiedlichen Wirkstoffen bzw. Wirkstoffkombinationen behandelt sind.

21. Verwendung einer Mikrotiterplatte mit einer Vielzahl von Wells für ein Verfahren zur Gasprobenentnahme bei der Untersuchung biologischer Systeme.

22. Verwendung nach Anspruch 21, **dadurch gekennzeichnet, dass** die verwendeten Wells eine Gehäusewandung aufweisen, die lichtdurchlässig ist.

23. Verwendung einer Mikrotiterplatte zur Durchführung eines Verfahrens nach einem der Ansprüche 1 bis 20.
